# EUROPEAN PATENT SPECIFICATION

(11) **EP 2 263 648 B1**
(45) Date of publication and mention of the grant of the patent: **08.04.2015**
(21) Application number: 10165945.6
(22) Date of filing: 15.06.2010
(51) Int. Cl.: A61K 31/714, A61K 31/728, A61K 31/185, A61K 9/00, A61P 27/02, A61K 45/06, A61K 9/08

(54) **Ophthalmic composition**
Ophthalmische Zusammensetzung
Composition ophtalmique

(30) Priority: 15.06.2009 IT RM20090102 U
(43) Date of publication of application: 22.12.2010
(73) Proprietor: Alfa Intes Industria Terapeutica Splendore S.r.l., Casoria (IT)
(72) Inventor: Gelsomino, Lucia, 80127 Napoli (IT)
(74) Representative: Bergadano, Mirko

(56) References cited:
- EP-A1- 2 002 841
- WO-A1-2008/001872
- FR-A1- 2 849 378

## Description

The present invention relates to an ophthalmic composition, in particular for stabilization of the lacrimal film, corneal cicatrization, restoration of the saline content of the tear, and osmoprotection, which contains a stabilized aqueous solution of cobalamine.

There are known and available on the market ophthalmic preparations with a base of cobalamine (vitamin B12), specifically cyanocobalamine, which would appear, however, to present the drawback of causing a troublesome burning sensation upon instillation.

It is on the other hand reported in the literature that cyanocobalamine decomposes when exposed to light and presents an optimal stability in aqueous solution at a pH of between 4.5 and 5.0. Said values fall outside or on the borderline with the range of pH tolerated by the eye, above all in the case of continuous and repeated administrations. On the other hand, at different values of pH cyanocobalamine is not stable, and a preparation containing cyanocobalamine would become ineffective.

FR2849378-A1 discloses an aqueous nutritive composition for use as an ophthalmological medicament comprising amino acids, vitamins, trace elements and metal salts and no cell growth factors, no animal or cell extracts and no drugs. In some embodiments, the composition comprises cyanocobalamine and sodium hyaluronate and has a pH of 7,3-7,5.

WO2008/001872 discloses an ophthalmic composition which contains alginic acid and/or a salt thereof in combination with hyaluronic acid and/or a salt thereof. In some embodiments, the composition further comprises cyanocobalamine and has a pH between 5 and 9.

There is not in practice available at the moment a cobalamine-based ophthalmic preparation that is not only effective, but is stable and does not cause burning sensations or irritation for the subject treated.

An aim of the present invention is to provide an ophthalmic composition containing cobalamine that is without the drawbacks of the known art presented above. In particular, an aim of the disclosure is to provide a composition that is fully effective in ophthalmology, in particular for stabilization of the lacrimal film, corneal cicatrization, restoration of the saline content of the tear, and osmoprotection, and is at the same time stable and presents a high ocular tolerability, i.e., it can be instilled in the eye, in drops, without practically causing any burning sensation upon instillation or with a burning sensation that is much less severe than known preparations.

The present invention hence relates to an ophthalmic composition, in particular for stabilization of the lacrimal film, corneal cicatrization, restoration of the saline content of the tear, and osmoprotection, as defined in essential terms in the annexed Claim 1 and, as regards its additional features, in the dependent claims.

In accordance with the invention, cobalamine (and specifically cyanocobalamine) is formulated at the lowest values of pH compatible with ocular administration, but with a stability sufficient for use and without presenting the drawback of the burning sensation upon instillation.

The composition according to the invention is hence characterized by a particular combination of active principles (mainly: cobalamine, hyaluronic acid and/or its salts, and also taurine) and excipients (such as, for example: sorbitol, trisodium citrate, pH-stabilizing substances, etc.), which, by acting synergistically, are able to stabilize chemically (cyano)cobalamine at a pH within the range 6.0 to 7.5, preferably 7.0 to 7.4, ensuring an excellent ocular tolerability.

The composition is in fact **characterized in that** it causes very little burning sensation or even no burning sensation at all (in particular at a pH of 7.0 to 7.4) following upon instillation of the product, a fact that is fundamental for patient compliance.

This fact is of extreme importance given that the patients for which the composition according to the invention is particularly indicated have in general irritated eyes and consequently feel any irritating factors to an amplified degree.

The composition according to the invention presents the following particularly favourable aspects:
1. Restoration of the saline content of the tear and osmoprotection.
   The composition according to the invention, thanks to the particular saline composition, and possibly to a slight hypotonia, is able to restore and keep at physiological levels the concentrations of important essential ions, such as sodium, potassium, and magnesium.
   The Na+/K+ molar ratio of the lacrimal liquid is physiologically approximately 5.5, whereas in dry-eye syndromes said ratio rises in favour of sodium up to values of 5.7 and beyond.
   The composition according to the invention is preferably formulated with a Na+/K+ ratio of approximately 5.20, which, together with the slight hypotonia of approximately 240 mOsm/L, is able to normalize, in the cases of "dry-eye", the levels of Na+ and K+ and the osmolarity at physiological values after just a few administrations.
2. Stabilization of the lacrimal film and corneal cicatrization.
   The composition according to the invention, thanks to the use of natural products, is able to stabilize the lacrimal film and to coadjuvate the physiological processes of tissue repair of the ocular surface.

In particular, cyanocobalamine, present physiologically in the aqueous humor, is able to coadjuvate corneal cicatrization and to stimulate proliferation of the corneal epithelium.

Sodium hyaluronate, thanks to its mucomimetic and pseudoplastic properties, is able to stabilize the lacrimal film by producing a protective viscoelastic coat that reduces friction and the stresses caused by ocular movements, thus accelerating healing of the surface keratites.

Taurine, which is normally present in the cornea, has proven effective in pre-clinical studies in favouring the normal processes of corneal cicatrization and reducing sub-epithelial haze following upon photokeratotomy by excimer laser.

The possible addition of aloe vera gel, a natural substance of vegetable origin, accelerates the times of corneal repair following upon photorefractive keratectomy with excimer laser in man in so far as it contains substances that have proven capable of coadjuvating sodium hyaluronate.

In practice, on the market, as in the literature, there do not exist other ophthalmic formulations that possess the particular balanced association of active principles (sodium hyaluronate, cyanocobalamine, taurine) and of excipients (sorbitol, trisodium citrate, NaCl, KCl, MgCl₂, pH-stabilizing agents, such as trometamol, sodium phosphates, etc.) present in the composition according to the invention.

Said substances, by co-operating synergistically, contribute to obtaining a formulation that is:
i) chemically stable;
ii) well tolerated at an ocular level;
iii) capable of stabilizing the lacrimal film and of normalizing its saline composition;
iv) suitable for coadjuvating corneal reparative processes.
The invention is further described in the following non-limiting examples of embodiment, which present respective formulations of compositions according to the invention.
In the various examples, the substances used for adjusting and stabilizing the pH of the compositions (trometamol, sodium phosphate dibasic dodecahydrate, etc.) are used in an amount such as to obtain compositions having a pH comprised between approximately 7.00 and approximately 7.20. Also provided is the value of pH actually detected.

**Example 1**

| Component | Quantity (mg/100 ml) |
|---|---|
| Sodium hyaluronate | 500 |
| Cyanocobalamine | 50 |
| Taurine | 500 |
| Aloe vera gel powder 200:1 | 5 ^{(*)} |
| Sorbitol | 500 |
| Trisodium citrate x 2 H₂O | 140 |
| NaCl | 400 |
| KCl | 100 |
| MgCl₂ x 6 H₂O | 25 |
| Trometamol | 7 |
| H₂O (w.f.i.) | as required to reach 100 |

| | |
|---|---|
| (*) 5 mg correspond to 1 g of fresh gel - pH: 7.15 - osmolarity: 243 mOsm/L | |

**Example 2**

| Component | Quantity (mg on 100 ml) |
|---|---|
| Sodium hyaluronate | 500 |
| Cyanocobalamine | 50 |
| Taurine | 500 |
| Sorbitol | 500 |
| Trisodium citrate x 2 H₂O | 140 |
| NaCl | 370 |
| KCl | 96 |
| MgCl₂ x 6 H₂O | 25 |
| Dibasic sodium phosphate dodecahydrate | 120 |
| Dibasic sodium phosphate dodecahydrate | 10 |
| H₂O (w.f.i.) | as required to reach 100 |

| | |
|---|---|
| - pH: 7.20 - osmolarity: 242 mOsm/L | |

Further compositions were prepared by varying the concentrations of the various components in the range summarized below:

**Further examples**

| Component | Quantity (mg on 100 ml) |
|---|---|
| Sodium hyaluronate | 400-600 |
| Cyanocobalamine | 40-60 |
| Taurine | 400-600 |
| Sorbitol | 400-600 |
| Trisodium citrate x 2 H₂O | 120-160 |
| NaCl | 300-400 |
| KCl | 80-120 |
| MgCl₂ x 6 H₂O | 20-30 |
| Dibasic sodium phosphate dodecahydrate | 100-150 |
| Dibasic sodium phosphate dodecahydrate | 5-15 |
| H₂O (w.f.i.) | as required to reach 100 |

| | |
|---|---|
| - pH: between 6.00 and 7.50 (preferably between approximately 7.15 and approximately 7.25) - osmolarity: between 220 and 260 mOsm/L - Na+/K+ molar ratio: between 5.0 and approximately 5.5 | |

It remains understood that the concentrations of the various components are provided purely by way of indicative example and may be varied in such a way that in any case the resulting aqueous solution has the values of pH indicated, at least between 6.00 and 7.50, preferably between 7.00 and 7.40, and more preferably still between approximately 7.0 and approximately 7.2.

Also the components indicated can be replaced by others, or else not be present.

For example, even though cyanocobalamine has principally been tested, other forms of cobalamine can be used; instead of or together with sodium hyaluronate other salts or derivatives of hyaluronic acid or the acid itself may be used; sodium, potassium, and magnesium can be added in other forms other than as chlorides; the pH-stabilizing substances can be different from the ones indicated.

Finally, it is understood that further modifications and variations may be made to the composition described herein, without thereby departing from the scope of the annexed claims.

## Claims

1. An ophthalmic composition, in particular for stabilization of the lacrimal film, corneal cicatrization, restoration of the saline content of the tear, and osmoprotection, **characterized by** comprising an aqueous solution containing cobalamine, hyaluronic acid and/or one or more salts of hyaluronic acid, and taurine, and one or more pH-stabilizing substances to keep the composition at a pH comprised between approximately 6.0 and approximately 7.5.

2. The composition according to Claim 1, having a pH comprised between approximately 7.00 and approximately 7.40.

3. The composition according to Claim 1 or Claim 2, wherein cobalamine is cyanocobalamine.

4. The composition according to any one of the preceding claims, containing sodium hyaluronate.

5. The composition according to any one of the preceding claims, containing sorbitol.

6. The composition according to any one of the preceding claims, wherein the pH-stabilizing substances are phosphates.

7. The composition according to any one of the preceding claims, wherein the stabilizing substances include sodium phosphate.

8. The composition according to any one of the preceding claims, containing salts, in particular chlorides of sodium, potassium and/or magnesium.

9. The composition according to any one of the preceding claims, having Na+/K+ molar ratio comprised between approximately 5.0 and approximately 5.5 and preferably around 5.20.

10. The composition according to any one of the preceding claims, having an osmolarity of between approximately 220 and approximately 260 mOsm/L.

11. The composition according to any one of the preceding claims, wherein cobalamine has a concentration of approximately 0.40 to 0.60 mg/ml.

12. The composition according to any one of the preceding claims, containing salt of hyaluronic acid at a concentration of approximately 4.00 to 6.00 mg/ml and taurine at a concentration of approximately 4.00 to 6.00 mg/ml.

13. The ophthalmic composition according to any one of the preceding claims, for use in stabilization of the lacrimal film, corneal cicatrization, restoration of the saline content of the tear, and osmoprotection.

## Patentansprüche

1. Eine ophthalmische Zusammensetzung, insbesondere zur Stabilisierung des Tränenfilms, Hornhautvernarbung, Wiederherstellung des Salzgehaltes der Tränen und Osmoprotektion, **dadurch gekennzeichnet, dass** sie eine wässrige Lösung, enthaltend Cobalamin, Hyaluronsäure und/oder ein oder mehrere Salze der Hyaluronsäure, und Taurin, und eine oder mehrere ph-stabilisierende Substanzen, um die Zusammensetzung bei einem pH zwischen ungefähr 6,0 und ungefähr 7,5 zu halten, umfasst.

2. Die Zusammensetzung gemäß Anspruch 1 mit einem pH zwischen ungefähr 7,00 und ungefähr 7,40.

3. Die Zusammensetzung gemäß Anspruch 1 oder Anspruch 2, wobei Cobalamin Cyanocobalamin ist.

4. Die Zusammensetzung gemäß einem der vorhergehenden Ansprüche, enthaltend Natriumhyaluronat.

5. Die Zusammensetzung gemäß einem der vorhergehenden Ansprüche, enthaltend Sorbitol.

6. Die Zusammensetzung gemäß einem der vorhergehenden Ansprüche, wobei die pHstabilisierenden Substanzen Phosphate sind.

7. Die Zusammensetzung gemäß einem der vorhergehenden Ansprüche, wobei die stabilisierenden Substanzen Natriumphosphat enthalten.

8. Die Zusammensetzung gemäß einem der vorhergehenden Ansprüche, enthaltend Salze, insbesondere Chloride von Natrium, Kalium und/oder Magnesium.

9. Die Zusammensetzung gemäß einem der vorhergehenden Ansprüche mit einem Na+/K+ Molverhältnis zwischen ungefähr 5,0 und ungefähr 5,5 und bevorzugt einem Na+/K+ Molverhältnis von etwa 5,20.

10. Die Zusammensetzung gemäß einem der vorhergehenden Ansprüche mit einer Osmolarität zwischen ungefähr 220 und ungefähr 260 mOsm/L.

11. Die Zusammensetzung gemäß einem der vorhergehenden Ansprüche, wobei Cobalamin eine Konzentration von ungefähr 0,40 bis 0,60 mg/ml aufweist.

12. Die Zusammensetzung gemäß einem der vorhergehenden Ansprüche, enthaltend ein Salz der Hyaluronsäure in einer Konzentration von ungefähr 4,00 bis 6,00 mg/ml und Taurin in einer Konzentration von ungefähr 4,00 bis 6,00 mg/ml.

13. Die ophthalmische Zusammensetzung gemäß einem der vorhergehenden Ansprüche zur Verwendung bei der Stabilisierung des Tränenfilms, Hornhautvernarbung, Wiederherstellung des Salzgehaltes der Tränen und Osmoprotektion.

## Revendications

1. Composition ophtalmique, en particulier pour la stabilisation du film lacrymal, la cicatrisation cornéenne, la restauration de la teneur en sels des larmes, et l'osmoprotection, **caractérisée en ce qu'**elle comprend une solution aqueuse contenant de la cobalamine, de l'acide hyaluronique et/ou un ou plusieurs sels de l'acide hyaluronique, et de la taurine, et une ou plusieurs substances de stabilisation du pH pour maintenir la composition à un pH compris entre environ 6,0 et environ 7,5.

2. Composition selon la revendication 1, ayant un pH compris entre environ 7,00 et environ 7,40.

3. Composition selon la revendication 1 ou la revendication 2, dans laquelle la cobalamine est de la cyanocobalamine.

4. Composition selon l'une quelconque des revendications précédentes, contenant du hyaluronate de sodium.

5. Composition selon l'une quelconque des revendications précédentes, contenant du sorbitol.

6. Composition selon l'une quelconque des revendications précédentes, dans laquelle les substances de stabilisation du pH sont des phosphates.

7. Composition selon l'une quelconque des revendications précédentes, dans laquelle les substances de stabilisation comprennent du phosphate de sodium.

8. Composition selon l'une quelconque des revendications précédentes, contenant des sels, en particulier des chlorures de sodium, de potassium et/ou de magnésium.

9. Composition selon l'une quelconque des revendications précédentes, présentant un rapport molaire Na+/K+ compris entre environ 5,0 et environ 5,5 et, de préférence, d'environ 5,20.

10. Composition selon l'une quelconque des revendications précédentes, ayant une osmolarité comprise entre environ 220 et environ 260 mOsm/L.

11. Composition selon l'une quelconque des revendications précédentes, dans laquelle la cobalamine a une concentration d'environ 0,40 à 0,60 mg/ml.

12. Composition selon l'une quelconque des revendications précédentes, contenant un sel d'acide hyaluronique à une concentration d'environ 4,00 à 6,00 mg/ml et de la taurine à une concentration d'environ 4,00 à 6,00 mg/ml.

13. Composition ophtalmique selon l'une quelconque des revendications précédentes, destinée à être utilisée pour la stabilisation du film lacrymal, la cicatrisation cornéenne, la restauration de la teneur en sels des larmes, et l'osmoprotection.
